(19) Europäisches Patentamt
European Patent Office
Office européen des brevets

(11) Veröffentlichungsnummer: 0 413 999 A2

# (12) EUROPÄISCHE PATENTANMELDUNG

(21) Anmeldenummer: 90114673.8

(22) Anmeldetag: 31.07.90

(51) Int. Cl.5: **C07D 251/46**, C07D 251/52, C07D 251/54

(30) Priorität: 22.08.89 DE 3927623

(43) Veröffentlichungstag der Anmeldung: 27.02.91 Patentblatt 91/09

(84) Benannte Vertragsstaaten: AT BE CH DE ES FR GB IT LI LU NL SE

(71) Anmelder: **HOECHST AKTIENGESELLSCHAFT**
Postfach 80 03 20
D-6230 Frankfurt am Main 80(DE)

(72) Erfinder: **Eberspach, Werner, Dr.**
Heimchenweg 84
D-6230 Frankfurt/Main 80(DE)
Erfinder: **Lenz, Günther**
Clarenbergweg 31
D-5020 Frechen(DE)
Erfinder: **Lysek, Manfred, Dr.**
Martin-Legros-Strasse 79
D-5300 Bonn(DE)

## (54) N,N'-Bis-1,3,5-triazin-6-yl-piperazine und Verfahren zu ihrer Herstellung.

(57) Die Erfindung betrifft neue N,N'-Bis-1,3,5-triazin-6-yl-piperazine der Formel

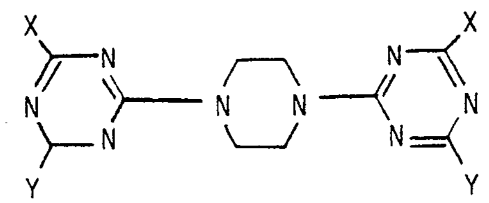

worin X und Y gleiche ouer verschiedene Reste $-OR^1$, $-SR^1$ oder $-NR^2R^3$ bedeuten wobei
$R^1$ für eine $C_1$- bis $C_{18}$-Alkylgruppe, eine $C_5$- bis $C_{18}$-Cycloalkylgruppe, eine Phenyl- oder Naphthylgruppe oder für eine $C_7$- bis $C_{18}$-Aralkylgruppe steht;
$R^2$ und $R^3$ unabhängig voneinander für eine $C_1$- bis $C_3$-Alkylgruppe, eine $C_5$- bis $C_{18}$-Cycloalkylgruppe, eine Phenyl- oder Naphthylgruppe oder für eine $C_7$- bis $C_{18}$-Aralkylgruppe stehen, oder $-NR^2R^3$ für Piperidinyl oder Morpholinyl steht.

Außerdem werden 2 Verfahren zur Herstellung der neuen Stoffe beschrieben.

EP 0 413 999 A2

# N,N′-BIS-1,3,5-TRIAZIN-6-YL-PIPERAZINE UND VERFAHREN ZU IHRER HERSTELLUNG

Die Erfindung betrifft neue N,N′-Bis-1,3,5-triazin-6-yl-piperazine der Formel

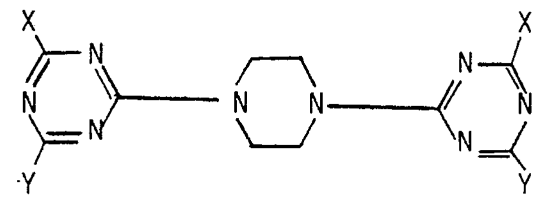

worin X und Y gleiche oder verschiedene Reste $-OR^1$, $-SR^1$ oder $-NR^2R^3$ bedeuten
wobei
$R^1$ für eine $C_1$- bis $C_{18}$-Alkylgruppe, eine $C_5$- bis $C_{18}$-Cycloalkylgruppe, eine gegebenenfalls mit inerten Resten substituierte Phenyl- oder Naphthylgruppe oder für eine $C_7$- bis $C_{18}$-Aralkylgruppe steht;
$R^2$ und $R^3$ unabhängig voneinander für eine $C_1$- bis $C_3$-Alkylgruppe, eine $C_5$- bis $C_{18}$-Cycloalkylgruppe, eine gegebenenfalls mit inerten Resten substituierte Phenyl- oder Naphthylgruppe oder für eine $C_7$- bis $C_{18}$-Aralkylgruppe stehen, oder
$-NR^2R^3$ für Piperidinyl der Formel

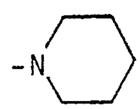

oder Morpholinyl der Formel

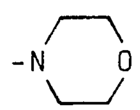

steht.

Als Amino-substituierte Triazinderivate sind die erfindungsgemäßen Verbindungen chemisoh verwandt mit bekannten Herbiziden wie 2-Ethylamino-4-tert.-butylamino-8-methoxy-1,3,5-triazin, 2-Methoxy-4,6-bis-(iso-propylamino)-1,3,5-triazin oder 2-Ethylamino-4-sec.-butylamino-8-methoxy-1,3,5-triazin und können wie diese als chemische Unkrautbekämpfungsmittel verwendet werden.

Ähnliche N,N′-Bis-1,3,5-triazin 6-yl-piperazine, die jedoch mindestens einen 2,2,6,6-Tetramethylpiperidylrest als Substituenten enthalten müssen und als Mittel zur Stabilisierung von Polymerisaten dienen sollen, sowie zwei verschiedene Analogieverfahren zu ihrer Herstellung wurden bereits in der DE 26 36 130 C3 beschrieben. Bei den beschriebenen Verfahren handelt es sich jeweils um zweistufige Verfahren mit Isolierung des Zwischenprodukts, oder es wird von einem bereits bekannten Zwischenprodukt ausgegangen, das in einstufiger Reaktion zum Zielprodukt umgesetzt wird.

In ähnlicher Weise betrifft auch die Erfindung zwei Verfahren zur Herstellung der neuen N,N′-Bis-1,3,5-triazin-6-yl-piperazine.

Beim Verfahren 1 wird in einem ersten Verfahrensschritt Cyanurhalogenid, vorzugsweise Cyanurchlorid, mit Piperazin zu N,N′-Bis(2,4-dichlor-1,3,5-triazin-6-yl)piperazin umgesetzt (vgl. DE 26 36 130 C3, Beispiel 6A). Im zweiten Verfahrensschritt entstehen die erfindungsgemäßen Bis-triazinyl-piperazine aus der Zwischenstufe durch Substitutionsreaktionen mit den Verbindungen XH und YH.

Der erste Verfahrensschritt kann beispielsweise so ausgeführt werden, daß Cyanurchlorid in einem geeigneten Suspensionsmittel vorgelegt wird und unter Kühlung gleichzeitig mit wässerigen Lösungen Piperazin sowie einer Base versetzt wird. Die Zugabe erfolgt derart, daß die Reaktionstem peratur zwischen -20 °C und 0 °C und der pH-Wert im Bereich von 5-7 gehalten werden kann. Das unlösliche Zwischenprodukt wird abfiltriert, mit Wasser gewaschen und in filterfeuchter Form for die 2. Stufe eingesetzt.

Abweichend von Beispiel 6A der DE 26 36 130 C3 führt man den ersten Reaktionsschritt vorzugsweise in einer 1:1-Mischung (Gew.-Teile) aus Eis und Aceton als Suspensionsmittel aus. Zum Abfangen des bei der Reaktion gebildeten Chlorwasserstoffs können Basen wie Hydroxide, Carbonate oder Hydrogencarbonate von Alkali- oder Erdalkalimetallen, vorzugsweise aber $Na_2CO_3$, eingesetzt werden. Das Molverhältnis der Base zu Cyanurchlorid beträgt (1,2 bis 1,0) : 1 bei einwertigen Basen wie NaOH und (0,6 bis 0,5) : 1 im

Falle von zweiwertigen Basen wie $Na_2CO_3$. Die Einsatzmenge des Piperazins beträgt 0,6 bis 0,5 Mol pro Mol Cyanurchlorid.

Der zweite Verfahrensschritt wird im allgemeinen in Abhängigkeit von der Verbindung XH und YH unterschiedlich ausgeführt.

Im Falle X = Y = $-OR^1$ suspendiert man das Zwischenprodukt z.B. in einem Überschuß des entsprechenden Alkohols, vorzugsweise Methanol oder Ethanol, oder in einer Mischung des Alkohols mit einem inerten Lösemittel und fügt 2,2 bis 2,0 Mol Base pro Mol Cyanurchlorid hinzu. Als Base eignen sich insbesondere Alkalihydroxide, die im entsprechenden Alkohol oder in einem inerten Lösemittel gelöst werden. Aufgrund der Schwerlöslichkeit des Zwischenproduktes ist es in einigen Fällen von Vorteil 0,01 bis 0.5 Gew% (bezogen auf Cyanurchlorid) eines Phasentransferkatalysators wie Tetra-n-butylammoniumhydrogensulfat als zusätzliche Komponente hinzuzufügen. Die Reaktionsmischung wird 12 bis 20 h unter Rückfluß erhitzt, anschließend abfiltriert und der Rückstand mit Wasser gewaschen und getrocknet.

Im Falle X = Y = $-SR^1$ oder $-NR^2R^3$ wird das filterfeuchte Zwischenprodukt in Wasser suspendiert und gleichzeitig mit Lösungen einer Base sowie des jeweiligen Thiols bzw. Amins versetzt. Als Lösemittel dient Wasser oder eine mit Wasser mischbare Flüssigkeit. Bei Raumtemperatur flüssige Thiole bzw. Amine können aber auch unverdünnt hinzugetropft werden. Geeignete Basen sind Hydroxide, Hydrogencarbonate oder Carbonate von Alkali- oder Erdalkalimetallen, welche in bezug auf in der ersten Stufe eingesetztes Cyanurchlorid im Molverhältnis (2,2 bis 2,0) : 1 (einwertige Basen) bzw. (1,1 bis 1,0) : 1 (zweiwertige Basen) eingesetzt werden. Das Molverhältnis des Thiols bzw. Amins zu in der ersten Stufe eingesetztem Cyanurchlorid beträgt (2,2 bis 2,0) : 1. Die Reaktionsmischung wird 12 bis 20 h unter Rückfluß erhitzt, anschließend filtriert und der Rückstand mit Wasser gewaschen und getrocknet.

Im Falle X ungleich Y lassen sich die erfindungsgemäßen N,N'-Bis-triazinyl-piperazine durch sukzessive Umsetzungen des Zwischenprodukts mit den Verbindungen XH und YH herstellen. Das Reaktionsprodukt der ersten Substitution wird abfiltriert, mit Wasser gewaschen und in filterfeuchter Form zur Zweitsubstitution eingesetzt. Die Reaktionsbedingungen der Erst- und Zweitsubstitution sind den bereits beschriebenen analoge

Im Falle X ungleich Y und X und/oder Y = $-OR^1$ beträgt das Molverhältnis der Base zum in der ersten Stufe eingesetzten Cyanurchlorid (1,1 bis 1,0) : 1 bei einwertigen Basen wie NaOH und (0,55 bis 0,5) : 1 bei zweiwertigen Basen wie $Na_2CO_3$.

Im Falle X ungleich Y und X und/oder Y = $-SR^1$ oder $-NR^2R^3$ beträgt das Molverhältnis des jeweiligen Thiols bzw. Amins zum in der ersten Stufe eingesetzten Cyanurchlorid (1,1 bis 1,0) : 1, das der Base (1,1 bis 1,0) : 1 bei einwertigen und (0,55 bis 0,5) : 1 bei zweiwertigen Basen.

Beim Verfahren 2 lassen sich die erfindungsgemäßen Verbindungen im Falle X = Y überraschenderweise in einer 2-Stufen-Eintopf-Synthese herstellen. Dabei wird zunächst das Cyanurhalogenid, vorzugsweise das Cyanurchlorid der Formel

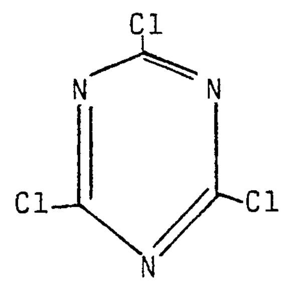

mit einer Verbindung XH oder YH in Gegenwart einer Base zum Abfangen des HCl zum Zwischenprodukt der Formel

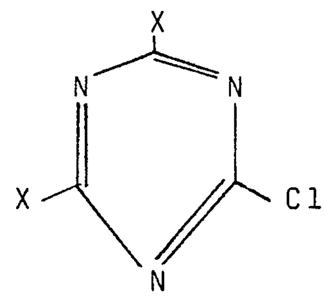

disubstituiert und dieses Zwischenprodukt ohne vorherige Isolierung im selben Reaktionsgefäß mit Piperazin der Formel

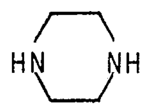 ,

wiederum in Gegenwart einer Base zum Abfangen des HCl, zum Zielprodukt umgesetzt

Man legt z.B. eine Mischung aus einer Base, der jeweiligen Verbindung XH oder YH sowie in einigen Fällen einem Phasentransferkatalysator vor und fügt Cyanurhalogenid in solcher Weise hinzu, daß die Reaktionstemperatur 30 °C nicht überschreitet und der pH-Wert zwischen 5 und 11 liegt. Die Reaktionstemperatur wird anschließend für 30 bis 40 min auf Siedetemperatur des Suspensionsmittels gehalten.

Im Falle X = Y = $-OR^1$ wird z.B. der entsprechende Alkohol, vorzugsweise Methanol oder Ethanol, im Überschuß eingesetzt, da er in Form einer Mischung aus 9-11 Vol.-Teilen Alkohol und 1 Vol.-Teil Wasser oder einer inerten Flüssigkeit gleichzeitig als Suspensionsmittel dient.

Im Falle X = Y = $-SR^1$ oder $-NR^2R^3$ wird Wasser oder eine inerte Flüssigkeit als Reaktionsmedium verwendet; das Molverhältnis des Thiols bzw. Amins zu Cyanurhalogenid beträgt dann (2,2 bis 2,0) : 1. Geeignete Basen sind Hydroxide, Carbonate oder Hydrogencarbonate von Alkali- oder Erdalkalimetallen. Das Molverhältnis der Base zu Cyanurhalogenid beträgt (2,2 bis 2,0) : 1 bei einwertigen und (1,1 bis 1,0) : 1 bei zweiwertigen Basen. Als Phasentransferkatalysator kann 0,1 bis 1 Gew% Tetra-n-butylammoniumhydrogensulfat, bezogen auf die eingesetzte Cyanurhalogenidmenge, verwendet werden

In der zweiten Stufe des Eintopf-Verfahrens fügt man der Reaktionsmischung wässerige Lösungen von Piperazin sowie einer Base hinzu und erhitzt für 2-18 h unter Rückfluß. Die im allgemeinen unlöslichen Bis-triazinyl-piperazine werden abfiltriert, mit Wasser gewaschen und getrocknet.

Das Molverhältnis von Piperazin zu in der 1. Stufe eingesetztem Cyanurhalogenid beträgt (1,2 bis 1,0) : 2. Als Basen eignen sich insbesondere $Na_2CO_3$ oder Alkalihydroxide, die in bezug auf in der 1. Stufe eingesetztes Cyanurhalogenid im Molverhältnis (0,6 bis 0,5) : 1 bzw. (1,2 bis 1,0) : 1 eingesetzt werden.

Im einzelnen betrifft die vorliegende Erfindung somit ein erstes Verfahren zur Herstellung der neuen N,N′-Bis-1,3,5-triazin-6-yl-piperazine, welches dadurch gekennzeichnet ist, daß man in einer ersten Reaktionsstufe Cyanurhalogenid, Piperazin sowie eine anorganische Base im Molverhältnis 1 : (0,5 bis 0,6) : (1 bis 1,2) im Falle einer einwertigen Base und im Molverhältnis 1 : (0,5 bis 0,6) : (0,5 bis 0,6) im Falle einer zweiwertigen Base in Gegenwart von Wasser und einem weiteren Suspensionsmittel bei Temperaturen von minus 20 °C bis 0 °C und bei einem pH-Wert von 5 bis 7 umsetzt und das als Zwischenprodukt erhaltene N,N′-Bis(2,4-dichlor-1,3,5-triazin-6-yl)-piperazin abfiltriert, mit Wasser wäscht und in einer zweiten Reaktionsstufe mit einer Verbindung XH oder YH und einer anorganischen Base im Molverhältnis (2 bis 2,2) : (2 bis 2,2) je Mol in die erste Reaktionsstufe eingesetztes Cyanurhalogenid im Falle einer einwertigen Base und im Molverhältnis (2 bis 2,2) : (1 bis 1,1) je Mol in die erste Reaktionsstufe eingesetztes Cyanurhalogenid im Falle einer zweiwertigen Base in Gegenwart eines Suspensionsmittels mischt 12 bis 20 Stunden unter Rückfluß erhitzt, abkühlt, neutralisiert und das Zielprodukt abfiltriert.

Das erste Herstellungsverfahren der Erfindung kann wahlweise und bevorzugt dadurch gekennzeichnet sein, daß man

a) in die zweite Reaktionsstufe einen Phasentransferkatalysator, vorzugsweise 0,01 bis 0,5 Gew% Tetra-n-butylammoniumhydrogensulfat, berechnet auf in der ersten Stufe eingesetztes Cyanurchlorid, zufügt;

b) in der ersten Reaktionsstufe nach Zugabe aller Reaktionskomponenten die Reaktionsmischung bis zu 1 h bei 0 °C nicht übersteigenden Temperaturen rührt;

c) im Falle X und/oder Y = $-OR^1$ in der zweiten Reaktionsstufe als Suspensionsmittel den entsprechenden Alkohol, das entsprechende Phenol oder Naphthol oder deren Mischung mit Wasser, Aceton,

Dioxan, Toluol oder Xylol einsetzt;

d) im Falle X und/oder Y = $-SR^1$ oder $-NR^2R^3$ in der zweiten Reaktionsstufe als Suspensionsmittel Wasser oder Gemische aus Wasser mit Aceton oder Dioxan einsetzt;

e) in der ersten Reaktionsstufe als weiteres Suspensionsmittel Aceton, Dioxan, Toluol oder Xylol einsetzt;

f) in der zweiten Reaktionsstufe im Falle X ungleich Y das Zwischenprodukt nacheinander mit den Verbindungen XH und YH in Gegenwart der anorganischen Base in den entsprechenden Molverhältnissen umsetzt, wobei das Reaktionsprodukt der ersten Substitution abfiltriert, mit Wasser gewaschen und in filterfeuchter Form zur Zweitsubstitution eingesetzt wird.

Die vorliegende Erfindung betrifft weiterhin ein zweites Verfahren zur Herstellung der neuen N,N′-Bis-1,3 5-triazin-6-yl-piperazine, welches dadurch gekennzeichnet ist, daß man im Rahmen einer Zweistufen-Eintopfreaktion in einer ersten Reaktionsstufe Cyanurhalogenid, eine Verbindung XH oder YH sowie eine anorganische Base im Molverhältnis 1 : (2 bis 2,2) : (2 bis 2,2) im Falle einer einwertigen Base und im Molverhältnis 1:(2 bis 2,2) : (1 bis 1,1) im Falle einer zweiwertigen Base in Gegenwart eines Suspensionsmittels bei einer 30 °C nicht überschreitenden Temperatur und einem pH-Wert von mindestens 5 zusemmengibt, die Reaktionsmischung bis zu 2 h bei Raumtemperatur rührt, anschließend 30 bis 40 Minuten unter Rückfluß erhitzt und in einer zweiten Reaktionsstufe die Reaktionsmischung mit Piperazin und einer anorganischen Base im Molverhältnis (0,5 bis 0,6) : (1 bis 1,2) je Mol in die erste Stufe eingesetztes Cyanurhalogenid im Falle einer einwertigen Base und im Molverhältnis (0,5 bis 0,6) : (0,5 bis 0,6) je Mol in die erste Stufe eingesetztes Cyanurhalogenid im Falle einer zweiwertigen Base in Gegenwart von Wasser rührt, 2 bis 18 h unter Rückfluß erhitzt, abkühlt, neutralisiert und das Zielprodukt abfiltriert.

Das zweite Herstellungsverfahren der Erfindung kann wahlweise und bevorzugt dadurch gekennzeichnet sein, daß man

g) in die erste Reaktionsstufe einen Phasentransferkatalysa tor, vorzugsweise 0,1 bis 1 Gew% Tetra-n-butylammoniumhydrogensulfat, berechnet auf eingesetztes Cyanurhalogenid, zufügt;

h) im Falle X = Y = $-OR^1$ als Suspensionsmittel eine Mischung des entsprechenden Alkohols, Phenols oder Naphthols $R^1OH$ mit Wasser, Aceton, Dioxan, Toluol oder Xylol einsetzt;

i) im Falle X = Y = $-SR^1$ oder $-NR^2R^3$ als Suspensionsmittel Wasser, Aceton, Dioxan, Toluol oder Xylol einsetzt und unter Stickstoffatmosphäre arbeitet;

J) als anorganische Basen Hydroxide, Hydrogencarbonate oder Carbonate von Alkali- oder Erdalkalimetallen einsetzt;

k) in der ersten Reaktionsstufe eine Mischung der Base, der Verbindung XH oder YH des Suspensionsmittels sowie gegebenenfalls des Phasentransferkatalysators vorlegt und Cyanurhalogenid so langsam zufügt, daß die Reaktionstemperatur 30 °C nicht überschreitet und der pH-Wert nicht unter 5 sinkt;

l) in der ersten Reaktionsstufe Cyanurhalogenid in einer Mischung aus Wasser und Eis suspendiert und die Verbindung XH oder YH sowie die Base so langsam einrührt, daß die Reaktionstemperatur 30 °C nicht überschreitet und der pH-Wert nicht unter 5 sinkt, und daß man die Mischung bis zu 2 h auf 70 bis 100 °C erhitzt und abkühlen läßt.

Die nachfolgenden Beispiele dienen der weiteren Erläuterung der beschriebenen Verfahren.

Beispiel 1 N,N′-Bis(2,4-di-methoxy-1,3,5-triazin-6-yl)piperazin

0,5 Mol Cyanurchlorid, 300 ml Aceton und 200 g Eis werden gemischt und bei minus 10 °C bis 0 °C sowie bei einem pH-Wert zwischen 5 und 7 gleichzeitig und tropfenweise mit je 0,25 Mol Piperazin und $Na_2CO_3$ als 8 gew%ige bzw. 20 gew%ige wässerige Lösungen versetzt. Man läßt 30 min bei 0 °C nachrühren, filtriert ab und wäscht mit Wasser. Der noch feuchte Filterkuchen wird in 0,2 l Methanol suspendiert und bei Raumtemperatur tropfenweise mit 1,05 Mol NaOH in Form einer 8 gew%igen methanolischen Lösung versetzt. Anschließend erhitzt man die Reaktionsmischung 18 h unter Rückfluß, läßt abkühlen und neutralisiert mit verdünnter $H_2SO_4$. Zuletzt wird abgenutscht, der Rückstand mit Wasser gewaschen und getrocknet. Man erhält ein feinkristallines, weißes Pulver.

Ausbeute: 92,3 % d. Th.

Schmelzpunkt: 230 - 233 °C (Zersetzung)

| | |
|---|---|
| Elementaranalyse: | gef. C 45,67 %, H 5,89 %, N 31,19 % |
| $C_{14}H_{20}N_8O_4$ (364,37) | ber. C 46,15 %, H 5,53 %, N 3o,75 % |

Beispiel 2 N,N′-Bis(2,4-di-ethoxy-1,3,5-triazin-6-yl)piperazin

0,5 Mol Cyanurchlorid werden in 300 ml Aceton vorgelegt und mit 200 g Eis versetzt. Anschließend fügt man 0,25 Mol Piperazin als 8 gew%ige wässerige Lösung und gleichzeitig 0,25 Mol $Na_2CO_3$ als 20 gew%ige wässerige Lösung hinzu und rührt 30 min nach. Während der Reaktion wird eine Temperatur von minus 15°C bis 0°C und ein pH-Wert von 5-7 eingehalten. Das Reaktionsprodukt wird abgenutscht, mit Wasser gewaschen und in filterfeuchter Form in 0,2 l Ethanol suspendiert. Bei Raumtemperatur gibt man nun 1,05 Mol KOH in Form einer 14 gew%igen ethanolischen Lösung tropfenweise hinzu und fügt noch 0,1 g Tetra-n-butylammoniumhydrogensulfat hinzu. Anschließend erhitzt man 15 h am Rückfluß, wobei gleichzeitig ein Teil des Ethanols abdestilliert. Zuletzt neutralisiert man mit verdünnter $H_2SO_4$, nutscht ab, wäscht mit Wasser und trocknet das weiße, feinkristalline Reaktionsprodukt bis zur Gewichtskonstanz.
Ausbeute: 87,4 % d. Th.
Schmelzpunkt: 190 - 193°C (Zersetzung)

| | |
|---|---|
| Elementaranalyse: | gef. C 50,95 %, H 6,35 %, N 27,03 % |
| $C_{18}H_{28}N_8O_4$ (420,48) | ber. C 51,42 %, H 6,71 %, N 26,65 % |

Beispiel 3 N,N′-Bis(2,4-di-morpholinyl-1,3,5-triazin-6-yl)piperazin

Sämtliche Arbeiten werden in einer $N_2$-Atmosphäre ausgeführt. 0,5 Mol Cyanurchlorid werden in 300 ml Aceton suspendiert und mit 200 g Eis versetzt. Anschließend tropft man gleichzeitig 0,25 Mol Piperazin als 13 gew%ige wässerige Lösung sowie 0,25 Mol $Na_2CO_3$ als 20 gew%ige wässerige Lösung zu. Dabei wird ein Temperaturbereich von minus 16°C bis minus 8°C und ein pH-Wert von 5-7 eingehalten. Nach dem Zutropfen wird 30 min bei minus 2°C bis minus 3°C nachgerührt, abfiltriert und der Filterrückstand mit Wasser gewaschen. Das filterfeuchte Reaktionsprodukt suspendiert man anschließend in 0,2 l Wasser und tropft bei Raumtemperatur gleichzeitig 1 Mol Morpholin sowie 1,05 Mol NaOH als 30 gew%ige wässerige Lösung hinzu. Die Reaktionsmischung wird 18 h unter Rückfluß erhitzt, mit verdünnter Schwefelsäure neutralisiert und abfiltriert. Zuletzt wäscht man mit Wasser und trocknet bis zur Gewichtskonstanz. Man erhält ein feinkristallines, weißes Pulver.
Ausbeute: 88,7 % d. Th
Schmelzpunkt: 318 - 321°C (Zersetzung)

| | |
|---|---|
| Elementaranalyse: | gef. C 53,06 %, H 7,25 %, N 29,21 % |
| $C_{26}H_{40}N_{12}O_4$ (584,69) | ber. C 53,41 %, H 6,89 %, N 28,75 % |

Beispiel 4 N,N′-Bis(2,4 di-piperidinyl-1,3,5-triazin-6-yl)piperazin

Alle Arbeiten werden in einer $N_2$-Atmosphäre ausgeführt. Zu einer Mischung aus 300 ml Aceton und 200 g Eis gibt man 0,5 Mol Cyanurchlorid und fügt gleichzeitig je 0,25 Mol Piperazin und $Na_2CO_3$ als 8 gew%ige bzw. 20 gew%ige wässerige Lösung hinzu. Die Reaktionstemperatur wird dabei zwischen minus 15°C und 0°C und der pH-Wert zwischen 5 und 7 eingehalten. Nach 30-minütigem Rühren wird die Reaktionsmischung abgenutscht, der Rückstand mit Wasser gewaschen und in 0,2 l Wasser suspendiert. Anschließend tropft man bei Raumtemperatur gleichzeitig 1 Mol Piperidin sowie 1,05 Mol NaOH als 30 gew%ige wässerige Lösungen hinzu und erhitzt 20 h unter Rückfluß. Die Reaktionsmischung wird abgenutscht, der Rückstand mit Wasser gewaschen und getrocknet. Man erhält ein feinkristallines, weißes Pulver.
Ausbeute: 97 % d. Th.
Schmelzpunkt: 305 - 307°C (Zersetzung)

| | |
|---|---|
| Elementaranalyse: | gef. C 62,08 %, H 8,75 %, N 29,60 % |
| $C_{30}H_{48}N_{12}$ (576,80) | ber. C 62,47 %, H 8,39 %, N 29,14 % |

Beispiel 5 N,N′-Bis(2,4-di-methoxy-1,3,5-triazin-6-yl piperazin

550 ml Methanol, 55 ml Wasser, 0,25 g Tetra-n-butylammoniumhydrogensulfat sowie 1 Mol $NaHCO_3$, werden vermischt und portionsweise mit 0,5 Mol Cyanurchlorid versetzt, so daß die Reaktionstemperatur 30° C nicht überschreitet und der pH-Wert zwischen 5 und 8 liegt. Man rührt 40 min bei Raumtemperatur nach und erhitzt 30 min unter Rückfluß. Anschließend werden bei Raumtemperatur gleichzeitig je 0,275 Mol Piperazin und $Na_2CO_3$ als 13 gew%ige bzw. 20 gew%ige wässerige Lösungen hinzugetropft und die Reaktionsmischung 1 h nachgerührt. Da nach erhitzt man 2 h unter Rückfluß, läßt abkühlen und neutralisiert mit verdünnter Schwefelsäure. Zuletzt wird filtriert, der Rückstand mit Wasser gewaschen und getrocknet. Man erhält ein feinkristallines, weißes Pulver.
Ausbeute: 87,2 % d. Th.
Schmelzpunkt: 229 - 232° C (Zersetzung)

| | |
|---|---|
| Elementaranalyse: | gef. C 45,78 %, H 5,7o %, N 31,18 % |
| $C_{14}H_{20}N_8O_4$ (364,37) | ber. C 46,15 %, H 5,53 %, N 3o,75 % |

Beispiel 6 N,N′-Bis(2,4-di-ethoxy-1,3,5-triazin-6-yl)piperazin

1 Mol $NaHCO_3$ und 0,5 g Tetra-n-butylammoniumhydrogensulfat werden in einer Mischung aus 400 ml Ethanol und 40 ml Wasser suspendiert und portionsweise so langsam mit 0,5 Mol Cyanurchlorid versetzt, daß die Reaktionstemperatur 30° C nicht überschreitet und der pH-Wert zwischen 5 und 8 liegt. Man rührt 60 min bei Raumtemperatur nach und erhitzt anschließend 30 min unter Rückfluß. Bei Raumtemperatur werden dann gleichzeitig je 0,275 Mol Piperazin und $Na_2CO_3$ als 12 gew%ige bzw. 20 gew%ige wässerige Lösungen tropfenweise hinzugefügt und die Reaktionsmischung 1 h nachgerührt. Anschließend wird 3 h unter Rückfluß erhitzt, bei Raumtemperatur abgenutscht und der Rückstand mit Wasser gewaschen und bis zur Gewichtskonstanz getrocknet. Man erhält ein feinkristallines, weißes Pulver.
Ausbeute: 81 % d. Th.
Schmelzpunkt: 189 - 193° C (Zersetzung)

| | |
|---|---|
| Elementaranalyse: | gef. C 5o,98 %, H 6,3o %, N 27,12 % |
| $C_{18}H_{28}N_8O_4$ (420,48) | ber. C 51,42 %, H 6,71 %, N 26,65 % |

Beispiel 7 N,N′-Bis(2,4-di-morpholinyl-1,3,5-triazin-6-yl)piperazin

Alle Arbeiten werden unter $N_2$ ausgeführt. Je 1 Mol $NaHCO_3$ und Morpholin werden mit 0,8 l Wasser gemischt und bei Raumtemperatur portionsweise so langsam mit 0,5 Mol Cyanurchlorid versetzt, daß die Reaktionstemperatur bei 25° C und der pH-Wert zwischen 7 und 10 liegt. Bei Raumtemperatur läßt man 100 min Nachrühren und erhitzt die Reaktionsmischung 40 min unter Rückfluß. Anschließend werden bei 25° C gleichzeitig je 0,262 Mol Piperazin und $Na_2CO_3$ als 13 gew%ige bzw. 20 gew%ige wässerige Lösungen unter Rühren hinzugetropft und der Ansatz 8 h unter Rückfluß erhitzt. Die abgekühlte Reaktionsmischung wird abfiltriert, der Rückstand mit Wasser gewaschen und bis zur Gewichtskonstanz getrocknet. Man erhält ein feinkristallines, weißes Pulver.
Ausbeute: 94,2 % d. Th.

Schmelzpunkt: 318 - 321 °C (Zersetzung)

| | |
|---|---|
| Elementaranalyse: | gef. C 52,92 %, H 7,11 %, N 29,14 % |
| $C_{26}H_{40}N_{12}O_4$ (584,69) | ber. C 53,41 %, H 6,89 %, N 28,75 % |

Beispiel 8 N,N′-Bis(2,4-di-piperidinyl-1,3,5-triazin-6-yl)piperazin

Sämtliche Arbeiten werden unter $N_2$-Überlagerung ausgeführt. Je 1 Mol $NaHCO_3$ und Piperidin werden in 0,8 l Wasser vorgelegt und bei Raumtemperatur portionsweise so langsam mit 0,5 Mol Cyanurchlorid versetzt, daß die Reaktionstemperatur bei 20 °C und der pH-Wert zwischen 7 und 11 liegt. Anschließend wird 100 min bei 20 °C nachgerührt und die Reaktionsmischung 30 min unter Rückfluß erhitzt. Nach dem Abkühlen fügt man gleichzeitig je 0,25 mol Piperazin und $Na_2CO_3$ als 13 gew%ige bzw. 20 gew%ige wässerige Lösungen hinzu, rührt 2 h bei Raumtemperatur nach und erhitzt 17 h unter Rückfluß. Die erhaltene Suspension wird abgenutscht, mit Wasser gewaschen und der Rückstand bis zur Gewichtskonstanz getrocknet. Man erhält ein feinkristallines, weißes Pulver.
Ausbeute: 93,3 % d. Th.
Schmelzpunkt: 303 - 307 °C (Zersetzung)

| | |
|---|---|
| Elementaranalyse: | gef. C 62,o1 %, H 8,87 %, N 29,45 % |
| $C_{30}H_{48}N_{12}$ (576,80) | ber. C 62,47 %, H 8,39 %, N 29,14 % |

Beispiel 9 N,N′-Bis(2,4-di-morpholinyl-1,3,5-triazin-6-yl)piperazin

Alle Arbeiten werden unter $N_2$ ausgeführt. 0,5 Mol Cyanurchlorid werden in einer Mischung aus 2000 ml Wasser und 1000 g Eis suspendiert und gleichzeitig tropfenweise mit 1,0 Mol Morpholin sowie 0,5 Mol NaOH als 10 gew%ige wässerige Lösung versetzt. Die Zugabe erfolgt so langsam, daß der pH-Wert zwischen 5 und 10 liegt. Anschließend läßt man 30 min bei 25 °C nachrühren und tropft weitere 0,5 Mol NaOH als 10 gew%ige wässerige Lösung hinzu. Die Reaktionsmischung wird 90 min auf 80 °C erhitzt und nach dem Abkühlen auf Raumtemperatur tropfenweise mit 0,28 Mol Piperazin und 0,56 Mol NaOH als 10 gew%ige wässerige Lösungen versetzt. Anschließend wird 12 h unter Rückfluß erhitzt, bei Raumtemperatur abgenutscht und der Rückstand mit Wasser gewaschen und getrocknet. Man erhält ein feinkristallines, weißes Pulver.
Ausbeute: 98,4 % d. Th.
Schmelzpunkt: 317 - 321 °C (Zersetzung)

| | |
|---|---|
| Elemantaranalyse: | gef. C 52,94 %, H 7,23 %, N 28,93 % |
| $C_{26}H_{40}N_{12}O_4$ (584,69) | ber. C 53,41 %, H 6,89 %, N 28,75 % |

Beispiel 10 N,N′-Bis(2-ethoxy-4-morpholinyl-1,3,5-triazin-6-yl)piperazin

Sämtliche Arbeiten werden in einer $N_2$-Atmosphäre ausgeführt. 0,5 Mol Cyanurchlorid werden in 300 ml Aceton vorgelegt und mit 200 g Eis versetzt. Anschließend fügt man 0,25 Mol Piperazin als 8 gew%ige wässerige Lösung und gleichzeitig 0,25 Mol $Na_2CO_3$ als 20 gew%ige wässerige Lösung hinzu und rührt 30 min nach. Während der Reaktion wird eine Temperatur von minus 15 °C bis 0 °C und ein pH-Wert von 5 - 7 eingehalten. Das Reaktionsprodukt wird abgenutscht, mit Wasser gewaschen und in filterfeuchter Form in 0,2 l Ethanol suspendiert. Bei Raumtemperatur tropft man nun 0,525 Mol KOH in Form einer 14 gew%igen

ethanolischen Lösung hinzu und fügt noch 0,1 g Tetra-n-butyl-ammoniumhydrogensulfat hinzu. Anschließend erhitzt man 10 h am Rückfluß, neutralisiert mit verdünnter $H_2SO_4$, nutscht ab und wäscht mit Wasser. Der filterfeuchte Rückstand wird in 0,2 l Wasser suspendiert und bei Raumtemperatur tropfenweise mit 0,5 Mol Morpholin sowie 0,525 Mol NaOH als 30 gew%ige wässerige Lösung versetzt. Anschließend erhitzt man 18 h unter Rückfluß, neutralisiert mit verdünnter $H_2SO_4$ und nutscht ab. Der Rückstand wird mit Wasser gewaschen und getrocknet. Man erhält ein feinkristallines, weißes Pulver.
Ausbeute: 85,7 % d. Th.
Schmelzpunkt: 220 - 223 °C (Zersetzung)

| | |
|---|---|
| Elementaranalyse: | gef. C 52,10 %, H 6,41 %, N 27,44 % |
| $C_{22}H_{34}N_{10}O_4$ (502,58) | ber. C 52,58 %, H 6,82 %, N 27,87 % |

Beispiel 11 N N′-Bis(2,4-di-α-naphthoxy-1,3,5-triazin-6-yl)piperazin

1 Mol $NaHCO_3$ und 0,5 g Tetra-n-butylammoniumhydrogensulfat werden in einer Mischung aus 1 Mol α-Naphthol und 400 ml Wasser suspendiert und portionsweise so langsam mit 0,5 Mol Cyanurchlorid versetzt, daß die Reaktionstemperatur 30 °C nicht überschreitet und der pH-Wert zwischen 5 und 8 liegt. Man rührt 60 min bei Raumtemperatur nach und erhitzt anschließend 40 min unter Rückfluß. Bei Raumtemperatur werden dann gleichzeitig je 0,275 Mol Piperazin und $Na_2CO_3$ als 12 gew%ige bzw. 20 gew%ige wässerige Lösungen tropfenweise hinzugefügt und die Reaktionsmischung 1 h nachgerührt. Anschließend wird 6 h unter Rückfluß erhitzt, bei Raumtemperatur abgenutscht und der Rückstand mit Wasser gewaschen und getrocknet. Man erhält ein feinkristallines, weißes Pulver.
Ausbeute: 82,0 % d. Th.
Schmelzpunkt: 211 - 213 °C (Zersetzung)

| | |
|---|---|
| Elementaranalyse: | gef. C 72,93 %, H 4,92 %, N 14,25 % |
| $C_{50}H_{36}N_8O_4$ (812,90) | ber. C 73,88 %, H 4,46 %, N 13,78 % |

**Ansprüche**

1. N,N′-Bis-1,3,5-triazin-6-yl-piperazine der Formel

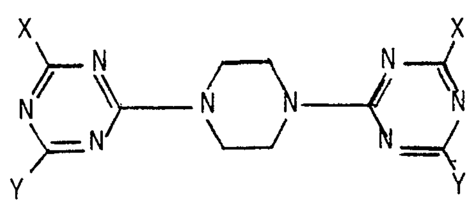

worin X und Y gleiche oder verschiedene Reste $-OR^1$, $-SR^1$ oder $NR^2R^3$ bedeuten,
wobei
$R^1$ für eine $C_1$- bis $C_{18}$-Alkylgruppe, eine $C_5$- bis $C_{18}$-Cycloalkylgruppe, eine gegebenenfalls mit inerten Resten substituierte Phenyl- oder Naphthylgruppe oder für eine $C_7$- bis $C_{18}$-Aralkylgruppe steht;
$R^2$ und $R^3$ unabhängig voneinander für eine $C_1$ bis $C_3$ -Alkylgruppe, eine $C_5$- bis $C_{18}$-Cycloalkylgruppe, eine gegebenenfalls mit inerten Resten substituierte Phenyl- oder Naphthylgruppe oder für eine $C_7$- bis $C_{18}$-Aralkylgruppe stehen, oder
$-NR^2R^3$ für Piperidinyl der Formel

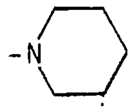

oder Morpholinyl der Formel

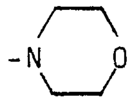

steht.

2. Verfahren zur Herstellung von N,N′-Bis-1,3,5-triazin-6-yl-piperazinen nach Anspruch 1, dadurch gekennzeichnet , daß man in einer ersten Reaktionsstufe Cyanurhalogenid, Piperazin sowie eine anorganische Base im Molverhältnis 1 : (0,5 bis 0,6) : (1 bis 1,2) im Falle einer einwertigen Base und im Molverhältnis 1 : (0,5 bis 0,6) : (0,5 bis 0,6) im Falle einer zweiwertigen Base in Gegenwart von Wasser und einem weiteren Suspensionsmittel bei Temperaturen von minus 20 °C bis 0 °C und bei einem pH-Wert von 5 bis 7 umsetzt und das als Zwischenprodukt erhaltene N,N′-Bis-(2,4-dichlor-1,3,5-triazin 6-yl)piperazin abfiltriert, mit Wasser wäscht und in einer zweiten Reaktionsstufe mit einer Verbindung XH oder YH und einer anorganischen Base im Molverhältnis (2 bis 2,2) : (2 bis 2,2) je Mol in die erste Reaktionsstufe eingesetztes Cyanurhalogenid im Falle einer einwertigen Base und im Molverhältnis (2 bis 2,2) : (1 bis 1,1) je Mol in die erste Reaktionsstufe eingesetztes Cyanurhalogenid im Falle einer zweiwertigen Base in Gegenwart eines Suspensionsmittels mischt, 12 bis 20 Stunden unter Rückfluß erhitzt, abkühlt, neutralisiert und das Zielprodukt abfiltriert.

3. Verfahren nach Anspruch 2, dadurch gekennzeichnet , daß man in die zweite Reaktionsstufe einen Phasentransferkatalysator, vorzugsweise 0,01 bis 0,5 Gew% Tetra-n-butylammoniumhydrogensulfat, berechnet auf in der ersten Stufe eingesetztes Cyanurhalogenid, zufügt.

4. Verfahren nach Anspruch 2 oder 3, dadurch gekennzeichnet , daß man in der ersten Reaktionsstufe nach Zugabe aller Reaktionskomponenten die Reaktionsmischung bis zu 1 h bei 0 °C nicht übersteigenden Temperaturen rührt.

5. Verfahren nach einem der Ansprüche 2 bis 4, dadurch gekennzeichnet , daß man im Falle X und/oder Y = $-OR^1$ in der zweiten Reaktionsstufe als Suspensionsmittel den entsprechenden Alkohol, das entsprechende Phenol oder Naphthol oder deren Mischung mit Wasser, Aceton, Dioxan, Toluol oder Xylol einsetzt.

6. Verfahren nach einem der Ansprüche 2 bis 4, dadurch gekennzeichnet , daß man im Falle X und/oder Y = $-SR^1$ oder $-NR^2R^3$ in der zweiten Reaktionsstufe als Suspensionsmittel Wasser oder Gemische aus Wasser mit Aceton oder Dioxan einsetzt.

7. Verfahren nach einem der Ansprüche 2 bis 8, dadurch gekennzeichnet , daß man in der ersten Reaktionsstufe als weiteres Suspensionsmittel Aceton, Dioxan, Toluol oder Xylol einsetzt.

8. Verfahren nach einem der Ansprüche 2 bis 7, dadurch gekennzeichnet , daß man in der zweiten Reaktionsstufe im Falle X ungleich Y das Zwischenprodukt nacheinander mit den Verbindungen XH und YH in Gegenwart der anorganischen Base in den entsprechenden Molverhältnissen umsetzt, wobei das Reaktionsprodukt der ersten Substitution abfiltriert, mit Wasser gewaschen und in filterfeuchter Form zur Zweitsubstitution eingesetzt wird.

9. Verfahren zur Herstellung von N,N′-Bis-1,3,5-triazin-6-yl-piperazinen nach Anspruch 1, dadurch gekennzeichnet , daß man im Rahmen einer Zweistufen-Eintopfreaktion in einer ersten Reaktionsstufe Cyanurhalogenid, eine Verbindung XH oder YH sowie eine anorganische Base im Molverhältnis 1 : (2 bis 2,2) : (2 bis 2,2) im Falle einer einwertigen Base und im Molverhältnis 1 : (2 bis 2,2) : (1 bis 1.1) im Falle einer zweiwertigen Base in Gegenwart eines Suspensionsmittels bei einer 30 °C nicht überschreitenden Temperatur und einem pH-Wert von mindestens 5 zusammengibt, die Reaktionsmischung bis zu 2 h bei Raumtemperatur rührt, anschließend 30 bis 40 Minuten unter Rückfluß erhitzt und in einer zweiten Reaktionsstufe die Reaktionsmischung mit Piperazin und einer anorganischen Base im Molverhältnis (0,5 bis 0,6) : (1 bis 1,2) je Mol in die erste Stufe eingesetztes Cyanurhalogenid im Falle einer einwertigen Base und im Molverhältnis (0,5 bis 0,6) : (0,5 bis 0,6) je Mol in die erste Stufe eingesetztes Cyanurhalogenid im Falle einer zweiwertigen Base in Gegenwart von Wasser rührt, 2 bis 18 h unter Rückfluß erhitzt, abkühlt, neutralisiert und das Zielprodukt abfiltriert.

10. Verfahren nach Anspruch 9, dadurch gekennzeichnet , daß man in die erste Reaktionsstufe einen Phasentransferkatalysator, vorzugsweise 0,1 bis 1 Gew% Tetra-n-butylammoniumhydrogensulfat, berechnet auf eingesetztes Cyanurhalogenid, zufügt.

11. Verfahren nach einem der Ansprüche 9 oder 10, dadurch gekennzeichnet , daß man im Falle X = Y = $-OR^1$ als Suspensionsmittel eine Mischung des entsprechenden Alkohols Phenols oder Naphthols $R^1$ OH mit Wasser, Aceton, Dioxan, Toluol oder Xylol einsetzt.

12. Verfahren nach einem der Ansprüche 9 oder 10, dadurch gekennzeichnet , daß man im Falle X = Y = $-SR^1$ oder $-NR^2R^3$ als Suspensionsmittel Wasser, Aceton, Dioxan, Toluol oder Xylol einsetzt und unter Stickstoffatmosphäre arbeitet.

13. Verfahren nach einem der Ansprüche 9 bis 12, dadurch gekennzeichnet , daß man als anorganische Basen Hydroxide, Hydrogencarbonate oder Carbonate von Alkali- oder Erdakalimetallen einsetzt.

14. Verfahren nach einem der Ansprüche 9 bis 13, dadurch gekennzeichnet , daß man in der ersten Reaktionsstufe eine Mischung der Base, der Verbindung XH oder YH, des Suspensionsmittels sowie gegebenenfalls des Phasentransferkatalysators vorlegt und Cyanurhalogenid so langsam zufügt, daß die Reaktionstemperatur 30 ° C nicht überschreitet und der pH-Wert nicht unter 5 sinkt.

15. Verfahren nach einem der Ansprüche 9 bis 13, dadurch gekennzeichnet , daß man in der ersten Reaktionsstufe Cyanurhalogenid in einer Mischung aus Wasser und Eis suspendiert und die Verbindung XH oder YH sowie die Base so langsam einrührt, daß die Reaktionstemperatur 30 ° C nicht überschreitet und der pH-Wert nicht unter 5 sinkt, und daß man die Mischung bis zu 2 h auf 70 bis 100 ° C erhitzt und abkühlen läßt.

Patentansprüche für folgenden Vertragsstaat: ES

1. Verfahren zur Herstellung von N,N′-Bis-1,3,5-triazin-6-yl-piperazinen der Formel

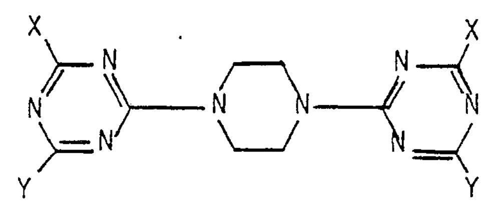

worin X und Y gleiche oder verschiedene Reste $-OR^1$, $-SR^1$ oder $-NR^2R^3$ bedeuten,
wobei
$R^1$ für eine $C_1$- bis $C_{18}$-Alkylgruppe, eine $C_5$-bis $C_{18}$-Cycloalkylgruppe, eine gegebenenfalls mit inerten Resten substituierte Phenyl- oder Naphthylgruppe oder für eine $C_7$- bis $C_{18}$-Aralkylgruppe steht;
$R^2$ und $R^3$ unabhängig voneinander für eine $C_1$- bis $C_3$-Alkylgruppe, eine $C_5$- bis $C_{18}$-Cycloalkylgruppe, eine gegebenenfalls mit inerten Resten substituierte Phenyl- oder Naphthylgruppe oder für eine $C_7$- bis $C_{18}$-Aralkylgruppe stehen, oder
$-NR^2R^3$ für Piperidinyl der Formel

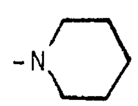

oder Morpholinyl der Formel

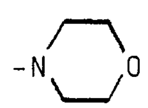

steht,

dadurch gekennzeichnet , daß man in einer ersten Reaktionsstufe Cyanurhalogenid, Piperazin sowie eine anorganische Base im Molverhältnis 1 : (0,5 bis 0,6) : (1 bis 1,2) im Falle einer einwertigen Base und im Molverhältnis 1 : (0,5 bis 0,6) : (0,5 bis 0,6) im Falle einer zweiwertigen Base in Gegenwart von Wasser und einem weiteren Suspensionsmittel bei Temperaturen von minus 20 ° C bis 0 ° C und bei einem pH-Wert von 5 bis 7 umsetzt und das als Zwischenprodukt erhaltene N,N′-Bis-(2,4-dichlor-1,3,5-triazin-6-yl)piperazin abfiltriert, mit Wasser wäscht und in einer zweiten Reaktionsstufe mit einer Verbindung XH oder YH und einer anorganischen Base im Molverhältnis (2 bis 2,2) : (2 bis 2,2) je Mol in die erste Reaktionsstufe eingesetztes Cyanurhalogenid im Falle einer einwertigen Base und im Molverhältnis (2 bis 2,2) : (1 bis 1,1) je Mol in die erste Reaktionsstufe eingesetztes Cyanurhalogenid im Falle einer zweiwertigen Base in Gegenwart eines Suspensionsmittels mischt, 12 bis 20 Stunden unter Rückfluß erhitzt, abkühlt, neutralisiert und das Zielprodukt abfiltriert.

2. Verfahren nach Anspruch 1, dadurch gekennzeichnet , daß man in die zweite Reaktionsstufe einen Phasentransferkatalysator, vorzugsweise 0,01 bis 0,5 Gew% Tetra-n butylammoniumhydrogensulfat, berechnet auf in der ersten Stufe eingesetztes Cyanurhalogenid, zufügt.

3. Verfahren nach Anspruch 1 oder 2, dadurch gekennzeichnet , daß man in der ersten Reaktionsstufe nach Zugabe aller Reaktionskomponenten die Reaktionsmischung bis zu 1 h bei 0 °C nicht übersteigenden Temperaturen rührt.

4. Verfahren nach einem der Ansprüche 1 bis 3, dadurch gekennzeichnet , daß man im Falle X und/oder Y = $OR^1$ in der zweiten Reaktionsstufe als Suspensionsmittel den entsprechenden Alkohol, das entsprechende Phenol oder Naphthol oder deren Mischung mit Wasser, Aceton, Dioxan, Toluol oder Xylol einsetzt.

5. Verfahren nach einem der Ansprüche 1 bis 3, dadurch gekennzeichnet , daß man im Falle X und/oder Y = $SR^1$ oder $-NR^2R^3$ in der zweiten Reaktionsstufe als Suspensionsmittel Wasser oder Gemische aus Wasser mit Aceton oder Dioxan einsetzt.

6. Verfahren nach einem der Ansprüche 1 bis 5, dadurch gekennzeichnet , daß man in der ersten Reaktionsstufe als weiteres Suspensionsmittel Aceton, Dioxan, Toluol oder Xylol einsetzt.

7. Verfahren nach einem der Ansprüche 1 bis 6, dadurch gekennzeichnet , daß man in der zweiten Reaktionsstufe im Falle X ungleich Y das Zwischenprodukt nacheinander mit den Verbindungen XH und YH in Gegenwart der anorganischen Base in den entsprechenden Molverhältnissen umsetzt, wobei das Reaktionsprodukt der ersten Substitution abfiltriert, mit Wasser gewaschen und in filterfeuchter Form zur Zweitsubstitution eingesetzt wird.

8. Verfahren zur Herstellung von N,N′-Bis-1,3,5-triazin-6-yl-piperazinen der Formel

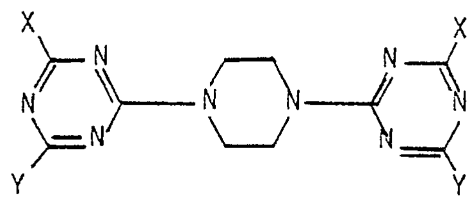

worin X und Y gleiche oder verschiedene Reste $-OR^1$, $-SR^1$ oder $-NR^2R^3$ bedeuten,
wobei
$R^1$ für eine $C_1$- bis $C_{18}$-Alkylgruppe, eine $C_5$-bis $C_{18}$-Cycloalkylgruppe, eine gegebenenfalls mit inerten Resten substituierte Phenyl- oder Naphthylgruppe oder für eine $C_7$-bis $C_{18}$-Aralkylgruppe steht;
$R^2$ und $R^3$ unabhängig voneinander für eine $C_1$- bis $C_3$-Alkylgruppe, eine $C_5$- bis $C_{18}$-Cycloalkylgruppe, eine gegebenenfalls mit inerten Resten substituierte Phenyl- oder Naphthylgruppe oder für eine $C_7$- bis $C_{18}$-Aralkylgruppe stehen, oder
$-NR^2R^3$ für Piperidinyl der Formel

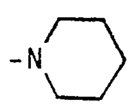

oder Morpholinyl der Formel

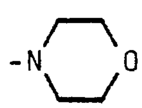

steht,

dadurch gekennzeichnet , daß man im Rahmen einer Zweistufen-Eintopfreaktion in einer ersten Reaktionsstufe Cyanurhalogenid , eine Verbindung XH oder YH sowie eine anorganische Base im Molverhältnis 1 : (2 bis 2,2) : (2 bis 2,2) im Falle einer einwertigen Base und im Molverhältnis 1 : (2 bis 2,2) : (1 bis 1,1) im Falle einer zweiwertigen Base in Gegenwart eines Suspensionsmittels bei einer 30 °C nicht überschreitenden Temperatur und einem pH-Wert von mindestens 5 zusammengibt, die Reaktionsmischung bis zu 2 h bei Raumtemperatur rührt, anschließend 30 bis 40 Minuten unter Rückfluß erhitzt und in einer zweiten Reaktionsstufe die Reaktionsmischung mit Piperazin und einer anorganischen Base im Molverhältnis (0,5 bis 0,6) : (1 bis 1,2) je Mol in die erste Stufe eingesetztes Cyanurhalogenid im Falle einer einwertigen Base und im Molverhältnis (0,5 bis 0,6) : (0,5 bis 0,6) je Mol in die erste Stufe eingesetztes Cyanurhalogenid im Falle einer zweiwertigen Base in Gegenwart von Wasser rührt, 2 bis 18 h unter Rückfluß erhitzt, abkühlt, neutralisiert und das Zielprodukt abfiltriert.

9 Verfahren naoh Anspruch 8, dadurch gekennzeichnet , daß man in die erste Reaktionsstufe einen Phasentransferkatalysator, vorzugsweise 0,1 bis 1 Gew% Tetra-n-butylammoniumhydrogensulfat, berechnet

auf eingesetztes Cyanurhalogenid, zufügt.

10. Verfahren nach einem der Ansprüche 8 oder 9, dadurch gekennzeichnet , daß man im Falle X = Y = $OR^1$ als Suspensionsmittel eine Mischung des entsprechenden Alkohols,Phenols oder Naphthols $R^1OH$ mit Wasser, Aceton, Dioxan, Toluol oder Xylol einsetzt.

11. Verfahren nach einem der Ansprüche 8 oder 9, dadurch gekennzeichnet , daß man im Falle X = Y = $SR^1$ oder $-NR^2R^3$ als Suspensionsmittel Wasser, Aceton, Dioxan, Toluol oder Xylol einsetzt und unter Stickstoffatmosphäre arbeitet.

12. Verfahren nach einem der Ansprüche 8 bis 11, dadurch gekennzeichnet , daß man als anorganische Basen Hydroxide, Hydrogencarbonate oder Carbonate von Alkali- oder Erdalkalimetallen einsetzt.

13. Verfahren nach einem der Ansprüche 8 bis 12, dadurch gekennzeichnet , daß man in der ersten Reaktionsstufe eine Mischung der Base der Verbindung XH oder YH, des Suspensionsmittels sowie gegebenenfalls des Phasentransferkatalysators vorlegt und Cyanurhalogenid so langsam zufügt, daß die Reaktionstemperatur 30 °C nicht überschreitet und der pH-Wert nicht unter 5 sinkt.

14. Verfahren nach einem der Ansprüche 8 bis 12, dadurch gekennzeichnet , daß man in der ersten Reaktionsstufe Cyanurhalogenid in einer Mischung aus Wasser und Eis suspendiert und die Verbindung XH oder YH sowie die Base so langsam einrührt, daß die Reaktionstemperatur 30 °C nicht überschreitet und der pH-Wert nicht unter 5 sinkt, und daß man die Mischung bis zu 2 h auf 70 bis 100 °C erhitzt und abkühlen läßt.